# EUROPÄISCHE PATENTANMELDUNG

(11) **EP 4 218 497 A1**
(43) Veröffentlichungstag der Anmeldung: **02.08.2023**
(21) Anmeldenummer: 22153850.7
(22) Anmeldetag: 28.01.2022
(51) Int. Cl.: A47D 13/02, A47D 13/08

(54) **LIEGEVORRICHTUNG FÜR DIE STABILE SEITENLAGERUNG EINES KINDES**

(71) Anmelder: Schroth, Caspar, 14109 Berlin (DE); Schroth, Katharina, 14109 Berlin (DE)
(72) Erfinder: Schroth, Katharina, 14109 Berlin (DE)
(74) Vertreter: Hertin und Partner Rechts- und Patentanwälte PartG mbB

(57) **Zusammenfassung**

Die Erfindung betrifft eine Liegevorrichtung (1), welche durch ihre Form eine stabile Seitenlagerung eines Kindes ermöglicht, wobei die Form einen Zentralbereich (3), und mindestens einen Kopfbereich (2) und/oder einen Fußbereich (4) umfasst, wobei der Zentralbereich einen geringeren Durchmesser als der Kopf- (2) und der Fußbereich (4) besitzt. In bevorzugten Ausführungsformen ist der geringere Durchmesser des Zentralbereichs (3) und/oder die mindestens eine Polstereinlage (6) im Zentralbereich (3) konfiguriert, um eine Bauch- oder Rückenlage des Kindes zu verhindern. In weiteren Ausführungsformen umfasst die Liegevorrichtung (1) mindestens eine Polstereinlage (6), welche an mindestens einer Innenwand (3a) im Zentralbereich (3) angebracht werden kann, um den Durchmesser (3b) des Zentralbereichs (3) zu verringern.

## Beschreibung

Die Erfindung betrifft eine Liegevorrichtung (1), welche durch ihre Form eine stabile Seitenlagerung eines Kindes ermöglicht, wobei die Form einen Zentralbereich (3), und mindestens einen Kopfbereich (2), und/oder einen Fußbereich (4) umfasst, wobei der Zentralbereich einen geringeren Durchmesser als der Kopf- (2) und der Fußbereich (4) besitzt. In bevorzugten Ausführungsformen ist der geringere Durchmesser des Zentralbereichs (3) und/oder die mindestens eine Polstereinlage (6) im Zentralbereich (3) konfiguriert, um eine Bauch- oder Rückenlage des Kindes zu verhindern. In Ausführungsformen umfasst die Liegevorrichtung (1) mindestens eine Polstereinlage (6), welche an mindestens einer Innenwand (3a) im Zentralbereich (3) angebracht werden kann, um den Durchmesser (3b) des Zentralbereichs (3) zu verringern.

### HINTERGRUND UND STAND DER TECHNIK

Das Schlafen in regulären Kleinkinderbetten birgt viele Nachteile für kleine Kinder, vor allem während des ersten Lebensjahres. Das Schlafen in der Bauchlage birgt für kleiner Kinder, besonders während des ersten Lebensjahres, eine erhöhte Gefahr des plötzlichen Kindstodes. Daher wird von Kinderärzten grundsätzlich für Neugeborene und Säuglinge das Schlafen in der Rückenlage empfohlen. Doch auch das Schlafen in der Rückenlage birgt in regulären Kinderbetten verschiedene Risiken. So kann bei Verwendung einer losen Bettdecke diese das Atmen des Kindes erschweren, wenn sie über den Kopf des Kindes rutscht. Auch Kopfkissen stellen in einem regulären Kinderbett ein Erstickungsrisiko dar und sind schlecht für die Entwicklung der Wirbelsäule.

Viele kleine Kinder schlafen zudem in der verordneten Rückenlage sehr schlecht, da diese, im Gegensatz zur engen Geborgenheit im Mutterleib, ungewohnten Bewegungsfreiraum und wenig Halt bietet, welche von Kindern im ersten Lebensjahr oft als störend empfunden wird. Vom Schlafen in der Seitenlage wird jedoch von Kinderärzten ebenfalls abgeraten, da sich das Kind leicht von der Seite auf den Bauch rollen kann, was wiederum das Risiko für den plötzlichen Kindstod erhöht.

Die Rückenlagerung von Kindern birgt zudem die Gefahr, dass sich der noch formbare Schädel von Säuglingen unter der ständigen Rückenlagerung abflacht. Diese Abflachung stellt eine pathologische Verformung des Schädels (Plagiozephalie) dar, welche teils mit sehr schmerzhaften und einschränkenden Therapiemaßnahmen, wie einem Helm, welcher den Schädel des Kindes in die gesunde Form zurückformt, behandelt wird.

Für eine Seitenlagerung von Säuglingen bietet der Stand der Technik ausschließlich verschiedene Kissen-Systeme an. Diese losen Kissen müssen jedoch in ein Kinderbett eingepasst werden und bieten womöglich erneut eine Erstickungsgefahr. Auch können diese Kissen im Kinderbett verrutschen, sodass das Kind trotzdem in die Bauchlage rollt. Diese teils sehr großen Seitenlagerungskissen-Systeme passen zudem selten in eine reguläre Baby-Tragetasche, was die Seitenlagerung eines Kindes während des Transportes schlicht verhindert, oder durch Verrutschen der Kissen in der engen Tragetasche ebenfalls die Atmung des Kindes behindern kann.

Eine sichere und für Kind und Eltern komfortable Lösung für die Seitenlagerung von Kindern, vor allem Neugeborenen und Säuglingen, ist somit mit den aus dem Stand der Technik bekannten Mitteln nicht möglich.

### ZUSAMMENFASSUNG DER ERFINDUNG

Aufgabe der vorliegenden Erfindung war es, die Nachteile des Standes der Technik zu beseitigen und eine verbesserte Liegevorrichtung für Kinder bereitzustellen, welche eine sichere und komfortable Seitenlagerung von Kleinkindern, vor allem Neugeborenen und Säuglingen, bietet, um die negativen Folgen einer Bauch-, und Rückenlagerung zu verhindern.

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

In einem Aspekt betrifft die Erfindung daher eine Liegevorrichtung (1), welche durch ihre Form eine stabile Seitenlagerung eines Kindes ermöglicht, wobei die Form einen Zentralbereich (3), und mindestens einen Kopfbereich (2), und/oder einen Fußbereich (4) umfasst, wobei der Zentralbereich einen geringeren Durchmesser als der Kopf- (2) und der Fußbereich (4) besitzt.

In einer Ausführungsform der Liegevorrichtung (1), welche durch ihre Form eine stabile Seitenlagerung eines Kindes ermöglicht, umfasst die Form einen Zentralbereich (3), einen Kopfbereich (2), und/oder einen Fußbereich (4), wobei der Zentralbereich einen geringeren Durchmesser als der Kopf- (2) und der Fußbereich (4) besitzt.

In manchen Ausführungsformen umfasst die Liegevorrichtung somit entweder einen Kopfbereich oder einen Fußbereich, jedoch immer einen Zentralbereich (3), dessen Durchmesser geringer ist als der des vorhandenen Kopf- und/oder Fußbereichs.

In einem Aspekt betrifft die Erfindung daher eine Liegevorrichtung (1), welche durch ihre Form eine stabile Seitenlagerung eines Kindes ermöglicht, wobei die Form einen Kopfbereich (2), einen Zentralbereich (3) und einen Fußbereich (4) umfasst, wobei der Zentralbereich einen geringeren Durchmesser als der Kopf- (2) und der Fußbereich (4) besitzt.

In spezifischen Ausführungsformen der Liegevorrichtung (1) ist die Umrandung bzw. der Rand des Kopf- und/oder des Fußbereiches unterbrochen, bzw. nicht durchgehend gestaltet, sodass die Liegevorrichtung zum Fuß- und/oder zum Kopfbereich der Liegevorrichtung und des darin liegenden Kindes hin "offen" bzw. nicht umrandet ist. Somit ähnelt in manchen Ausführungsformen die Liegevorrichtung in der Aufsicht eher einer (nach oben und/oder unten hin offenen) Sanduhr als einer geschlossenen 8-Form. Diese spezifischen Ausführungsformen ermöglichen eine sehr variable Anpassung der erfindungsgemäßen Liegevorrichtung an die Körperhöhe eines Kindes und dennoch eine stabile Seitenlagerung.

In manchen Ausführungsformen hat der Rand in unterschiedlichen Bereichen der Liegevorrichtung eine unterschiedliche Höhe.

In Ausführungsformen besitzt somit der Rand der Liegevorrichtung (1) eine unterschiedliche Höhe im Zentralbereich (3), Kopfbereich (2) und/oder Fußbereich (4) oder ist im Kopf- und/oder Fußbereich unterbrochen.

Bevorzugt ist die erfindungsgemäße Liegevorrichtung dazu eingerichtet eine Seitenlagerungsfixierung für Kinder zu gewährleisten. In Ausführungsformen wird das Kind seitlich in die Liegevorrichtung (1) gelegt. Dabei wird der Rumpf des Kindes durch den Zentralbereich so fixiert, dass sich das Kind weder auf den Rücken noch auf den Bauch drehen kann. Somit wird insbesondere ein Vorrollschutz sichergestellt. In Ausführungsformen wird das seitlich in die Liegevorrichtung hineinzulegte Kind am Rumpf durch den Zentralbereich, und optional durch dort zusätzlich angebrachte Polstereinlagen, fixiert, während die Kopf- und Beinpartie Bewegungsfreiheit besitzen.

Die Erfinderin hat überraschenderweise festgestellt, dass die Seitenlagerungsfixierung in der erfindungsgemäßen Liegevorrichtung auf darin liegende Kinder unerwartet beruhigend wirkt. Dieser Effekt wird durch die ungewöhnliche Form der erfindungsgemäßen Liegevorrichtung erreicht, wobei die Kinder eine beruhigende und enganliegende Fixierung im Bereich des Torsos erfahren, welche zugleich eine Bauch- oder Rückenlage verhindert. Zusätzlich ermöglicht die größere Bewegungsfreiheit des Kopfes und Fußbereiches eine uneingeschränkte Bewegung und wunschgemäße Lagerung der Gliedmaßen und des Kopfes des Kindes in der Seitenlage.

Durch die Kombination eines großen Luftvolumens im Kopfbereich und der fixierenden Taillierung der Liegevorrichtung im Zentralbereich, welche eine stabilisierte Seitenlage bewirkt, kann die erfindungsgemäße Liegevorrichtung zusätzlich zum gesteigerten Wohlbefinden des Kindes auch den überraschenden Effekt erzielen, dass zum einen eine Bauchlage des Kindes verhindert wird, in welcher eine erhöhte Wahrscheinlichkeit des plötzlichen Kindstods bestehen kann. Zugleich kann auch eine Rückenlage des Kindes vermieden werden, welche zu einer Abflachung des Hinterkopfes führen kann. Das Schlafen in der Bauchlage wird bei Kindern im ersten Lebensjahr als Risikofaktor für den plötzlichen Kindstod angesehen. In der Prophylaxe des plötzlichen Kindstods wird seit Jahrzehnten als einer der wichtigsten Faktoren die Rückenlage, für Kleinkinder und Neugeborene im ersten Lebensjahr während des Schlafes empfohlen. Die Rückenlagerung gilt als zuverlässige Methode, da Neugeborene sich vor dem sechsten Lebensmonat in der Regel nicht selbstständig drehen können, sodass die angeratene Rückenlage das Hinüberrollen in die Bauchlage verhindert. Kinder unter einem Jahr können sich meist noch nicht stabil in der Seitenlage halten, sodass beim Schlaf in der Seitenlage immer die Gefahr des Rollens in die Bauchlage besteht.

Leider hat die frühe Rückenlagerung auch negative Folgen für manche Kinder. Zum einen schlafen kleine Kinder, vor allem Neugeborene, deutlich schlechter auf dem Rücken, da die Neugeborenen nach 9 Monaten des geborgenen ,Gepucktseins' (eng-umschlossen-seins) in Embryostellung im Mutterleib nicht gut mit dem plötzlichen Freiraum umgehen können. Da den Neugeborenen nach der Geburt die Begrenzung und der Kontakt vor allem an der Körpermitte und Vorderseite fehlen, wird der reguläre Schlaf in der Rückenlage häufig durch einschießende Muskelzuckungen (Myoklonien) und Motoreflexe gestört. Durch die erhöhte Unruhe werden Wirbelbildungen im Darm und damit Blähungen begünstigt. Ein weiterer negativer Effekt der erzwungenen Rückenlage bei Neugeborenen und Kleinkindern ist die durch die Rückenlage verursachte Abflachung bzw. Verformung des Hinterkopfes (Plagio-zephalus), den man - einmal geschehen - nur noch durch strikte Lagerung, Krankengymnastik oder in schweren Fällen durch eine äußerst schmerzhafte Helmtherapie behandeln kann.

In einer weiteren bevorzugten Ausführungsform der Erfindung enthält die Form mindestens eine Polstereinlage (6), welche an mindestens einer Innenwand (3a) im Zentralbereich (3) angebracht werden kann, um den Durchmesser (3b) des Zentralbereichs (3) zu verringern.

In einer weiteren bevorzugten Ausführungsform der Erfindung sind der geringere Durchmesser des Zentralbereichs (3) und/oder die mindestens eine Polstereinlage (6) im Zentralbereich (3) konfiguriert, um eine Bauch- oder Rückenlage des Kindes zu verhindern.

Da Neugeborene, Kleinkinder und Kinder unterschiedlich groß sind und wachsen, kann in spezifischen Ausführungsformen der Liegevorrichtung innen an der mindesten einen Polstereinlage (6) im Zentralbereich jeweils mindestens eine zusätzliche Polstereinlage (6) angebracht werden, um den Durchmesser (3b) des Zentralbereiches anzupassen (mehrere Polstereinlagen können optional übereinander angebracht werden, um den Durchmesser des Zentralbereiches schrittweise zu verringern/vergrößern). Auf diese Weise können Ausführungsformen der Liegevorrichtung sowohl für Neugeborene als auch für größere Kleinkinder verwendet werden, sodass sich eine Liegevorrichtung im Laufe des Wachstums des Neugeborenen an dessen Größe anpassen lässt. Dieses hat zum einen den Vorteil, dass zu jedem Wachstumsstadium das Neugeborene bzw. (Klein)Kind optimal in der Liegevorrichtung in der Seitenlage fixiert ist, ohne dass das Wohlbefinden des Kindes eingeschränkt ist. Außerdem bewirkt diese flexible Größenanpassung, dass Kosten und Ressourcen eingespart werden, da keine neue Liegevorrichtung erworben werden muss, wenn das Neugeborene oder Kleinkind wächst.

Durch die flexible Größenanpassung des Zentralbereiches sind verschiedene Ausführungsformen für die Fixierung des Kindes möglich: z.B. sind Ausführungsformen 1. ohne Hinzufügung einer Polstereinlage im Zentralbereich für größere (Klein)Kinder möglich, als auch z.B. 2. unter Hinzufügung von mindesten einer Polstereinlage im Zentralbereich und z.B. 3. unter Hinzufügung von mindesten einer zusätzlichen Polstereinlage auf der Innenseite einer bereits angebrachten Polstereinlage (sodass zwei Polstereinlagen übereinander angebracht sind). So kann durch das Hinzufügen zusätzlicher Polstereinlagen an die Innenwände des Zentralbereichs der Liegevorrichtung der Durchmesser des Zentralbereichs, welcher den Torso des Kindes in der Seitenlage fixiert, schrittweise vergrößert oder verringert werden, sodass sich die Liegevorrichtung an die Größe des Kindes individuell anpassen kann. Die Polstereinlagen können in einer Ausführungsform abgeklappt und /oder als Einzelteile angebracht werden.

Die Fixierung der Polstereinlagen im Zentralbereich kann in Ausführungsformen der Erfindung auf verschiedene Weise erfolgen, wobei die Polstereinlagen (6) wahlweise an der Innenwand (3a) des Zentralbereiches oder auf einer bereits im Zentralbereich angebrachten Polstereinlage (6) angebracht werden können. Die Mittel zur Fixierung der Polstereinlage sind in Ausführungsformen ausgewählt aus der Gruppe umfassend Klettverschluss, Haken, Druckknöpfe, Knöpfe, Reißverschluss, Ösen, Gurte, Schlaufen, Anti-rutsch-Materialien (z.B. Gummi), durch Einschub in eine an der Wand des Zentralbereiches angenähte/angebrachte Tasche und/oder Schlaufe und jede mögliche Kombination dieser Mittel.

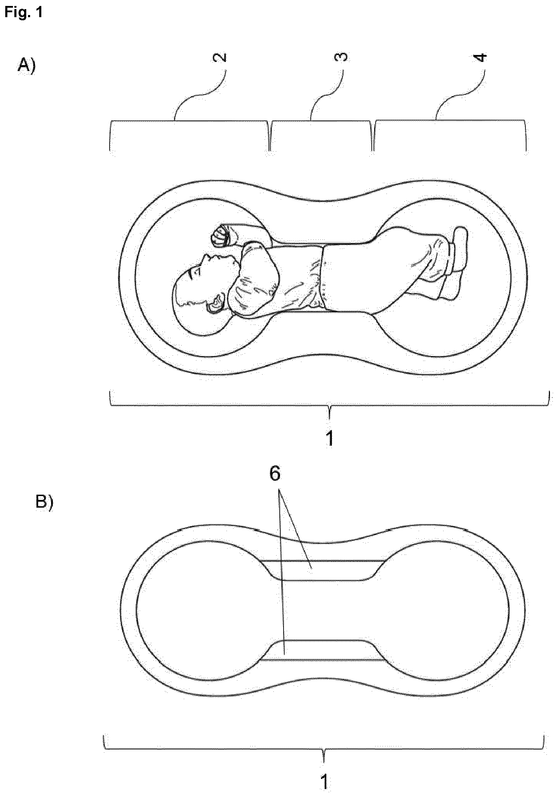

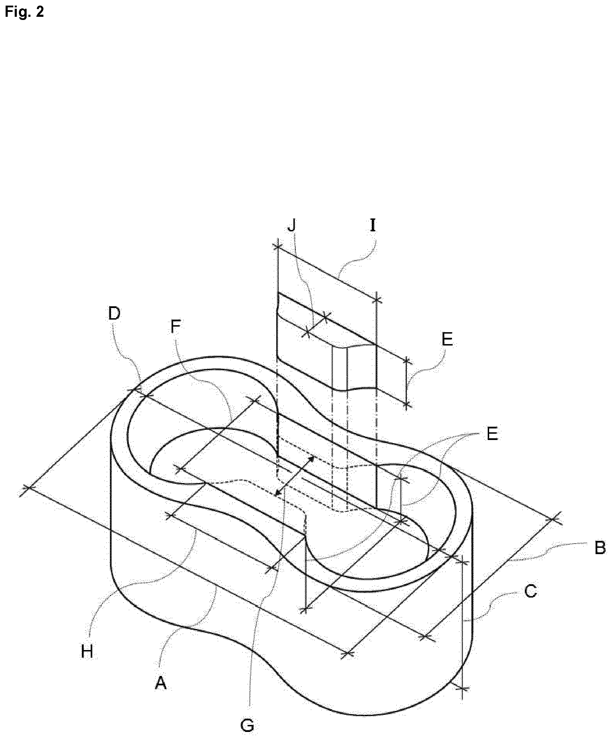

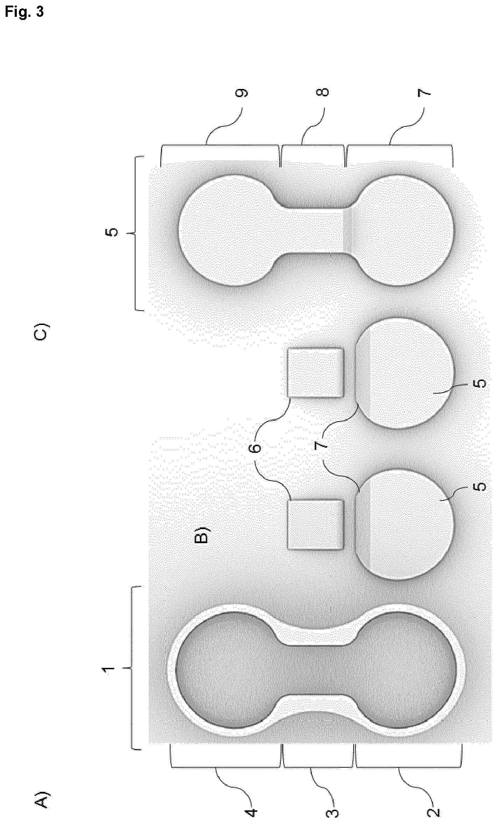

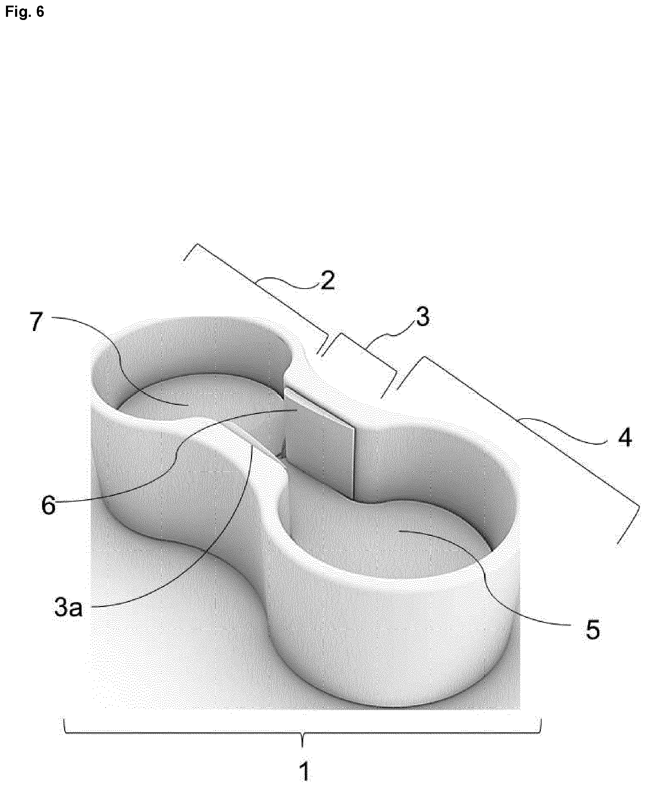

In manchen Ausführungsformen der Liegevorrichtung (1) ist die mindestens eine Polstereinlage (6) keilförmig gestaltet (siehe z.B. Figuren 3 und 6). In spezifischen Ausführungsformen der Liegevorrichtung ist die mindestens eine Polstereinlage zur Innenseite hin gerade geschnitten, so dass "Schienen" entstehen, die den Rumpf des Kindes in Seitenlage fixieren (Siehe Figuren 1B und 2). In Ausführungsformen sind andere Formen der Polstereinlage ebenfalls möglich, wobei unterschiedlich geformte Polstereinlagen in der Liegevorrichtung auch kombiniert werden können. In bestimmten Ausführungsformen ist die gesamte Konstruktion der Liegevorrichtung aus einem Stück gefertigt, so dass die Polstereinlagen, die für die Fixierung des Kindes in Seitenlage verantwortlich sind, feststehen und damit selbst fixiert sind. In anderen Ausführungsformen ist die Liegevorrichtung aus mehr als einem Stück gefertigt, so dass einzelne Komponenten auswechselbar und/oder herausnehmbar sind.

In Ausführungsformen der Erfindung umfasst die Liegevorrichtung (1) mindestens eine Matratzeneinlage (5), wobei vorzugsweise die mindestens eine Matratzeneinlage (5) im Kopfbereich (7) dicker ist als im Zentral- (8) und Fußbereich (9).

In Ausführungsformen der Liegevorrichtung (1) wird mindestens eine Matratze (5) oder Matratzeneinlage (5) eingelegt, die in bevorzugten Ausführungsformen am Kopf leicht erhöht ist, um den in der Seitenlagerung des Kindes untenliegenden Arm zu entlasten. In manchen Ausführungsformen besitzt die Matratze (5) oder Matratzeneinlage (5) einen dickeren/erhöhten Kopfbereich, der als Kissen dient, in anderen Ausführungsformen umfasst die Matratze ein separates Kopfkissen. Die mindestens eine Matratze (5) oder Matratzeneinlage (5) kann in verschiedenen Ausführungsformen z.B. für kleine Babys mit nur leichter Kopferhöhung oder für größere Babys mit etwas stärkeren Kopferhöhung ausgefertigt sein. Andere Ausführungsformen sind auch gewünscht. In verschiedenen Ausführungsformen besitzt das Kopfkissen eine bestimmte Dicke. So können z.B. für größere Kinder Kopfkissen mit einer größeren Dicke verwendet werden. Vorzugsweise können Kopfkissen mit unterschiedlichen Dicken/Höhen mit einer Matratze kombiniert werden, was eine zusätzliche individuelle Anpassung der Liegevorrichtung an das Kind und dessen Wachstum ermöglicht.

In einer weiteren Ausführungsform besitzt die Matratze (5) oder Matratzeneinlage (5) eine verringerte Dicke im Kopfbereich (7), wobei vorzugsweise in diesen Ausführungsformen ein zusätzliches Kopfkissen (7) verwendet werden kann. In einer weiteren Ausführungsform besitzt die mindestens eine Matratze eine geringere Dicke im Kopfbereich (7), sodass ein zusätzlich verwendetes Kissen im Kopfbereich (7) der Matratze (5) und der Liegevorrichtung fixiert wird und ein Verrutschen des Kissens, z.B. nach unten hin, verhindert wird. In spezifischen Ausführungsformen besteht die Matratze (5) oder Matratzeneinlage (5) nur aus einem Kissen bzw. Kopfteil (7).

In einer alternativen Ausführungsform besitzt die mindestens eine Matratze eine einheitliche Dicke im Kopfteil (7), Zentralteil (8) und Fußteil (9).

In einer weiteren Ausführungsform der erfindungsgemäßen Liegevorrichtung (1) besteht die mindestens eine Matratzeneinlage (5) aus einem verbundenen Teil oder aus mehreren separaten oder verbundenen Teilen besteht, umfassend ein Zentralteil (8), ein Kopfteil (7) und/oder ein Fußteil (9), wobei die separaten oder verbundenen Teile der Matratzeneinlage (5) eine unterschiedliche oder die gleiche Dicke aufweisen.

In einer weiteren Ausführungsform der erfindungsgemäßen Liegevorrichtung (1) besteht die mindestens eine Matratzeneinlage (5) aus einem verbundenen Teil oder aus mehreren separaten oder verbundenen Teilen, umfassend ein Kopfteil (7), ein Zentralteil (8) und ein Fußteil (9), wobei die separaten oder verbundenen Teile der Matratzeneinlage (5) eine unterschiedliche oder die gleiche Dicke aufweisen.

Eine Matratzeneinlage oder Matratze kann in Ausführungsformen zwischen 1 und 15 cm dick sein, zwischen 1 und 10 cm, oder zwischen 2 und 7 cm. Wenn in Ausführungsformen die Verwendung eines zusätzlichen Kopfkissens angedacht ist oder wenn die Matratze eine einheitliche Dicke aufweist ist sie vorzugsweise zwischen 2 und 5 cm dick. In Ausführungsformen ist die Dicke der Matratze auch von der Festigkeit ihres Materials abhängig. Um einen optimalen Liegekomfort zu ermöglichen, sind Matratzen aus weicheren Materialien vorzugsweise dicker als Matratzen aus festeren Materialien, in welche das Kind nicht so tief einsinkt. In manchen Ausführungsformen ist das Kopfkissen aus dem gleichen Material, wie die Matratze gefertigt. In alternativen Ausführungsformen ist das Kopfkissen aus einem anderen Material und/oder einem Material mit einer anderen Festigkeit als die restliche Matratzeneinlage gefertigt.

In manchen Ausführungsformen ist die Matratzeneinlage mit einem wasserabweisenden oder wasserdichten Material oder Imprägnierung überzogen, um eine Verschmutzung der Matratze zu verhindern. In manchen Ausführungsformen kann die Matratze zusätzlich an der Liegevorrichtung fixiert werden. Die Mittel zur Fixierung der Matratze sind in manchen Ausführungsformen ausgewählt aus der Gruppe umfassend Klettverschluss, Haken, Druckknöpfe, Knöpfe, Anti-rutsch-Materialien (z.B. Gummi), Reißverschluss, Ösen, Gurte oder jegliche Kombination dieser Fixierungen.

In einer weiteren Ausführungsform der erfindungsgemäßen Liegevorrichtung (1) ist das Kind vorzugweise ein Neugeborenes oder ein Säugling. In einer weiteren Ausführungsform der erfindungsgemäßen Liegevorrichtung (1) ist das Kind ein Kleinkind unter 3 Jahren.

In Ausführungsformen der erfindungsgemäßen Liegevorrichtung (1) besitzt die Liegevorrichtung (1) die äußeren Dimensionen (Breite × Länge) von 25-50 × 60-100 cm, vorzugsweise von 30-40 × 65-85 cm, wobei die Außenwände der Liegevorrichtung (1) zwischen 15-30 cm hoch sind, vorzugsweise zwischen 20-35 cm hoch.

In Ausführungsformen der Erfindung haben die Abschnitte bzw. Teile der erfindungsgemäßen Liegevorrichtung (1) definierte Dimensionen. Die Dimensionen spezifischer Ausführungsformen der Erfindung sind in Figur 2 dargestellt.

Die hierin definierten Dimensionen **A-J** sind als voneinander separate und unabhängige Ausführungsformen der erfindungsgemäßen Liegevorrichtung zu verstehen, und bestehen in keiner bestimmten oder zwangsläufigen Kombination miteinander. Entsprechend kann in Ausführungsformen der Liegevorrichtung jeder spezifischer Wert einer Dimension mit jedem Wert anderer Dimensionen kombiniert werden, oder allein für eine Ausführungsform bestehen. In anderen Worten, in spezifischen Ausführungsformen der erfindungsgemäßen Liegevorrichtung haben Teile der erfindungsgemäßen Liegevorrichtung (1) die folgenden Dimensionen, wobei die Dimensionen verschiedener Teile nicht zwangsläufig miteinander kombiniert sind und in Ausführungsformen einzeln vorkommen oder unabhängig voneinander vorliegen oder kombiniert werden können.

**A** bezieht sich auf die Gesamtlänge der Liegevorrichtung (1). In Ausführungsformen der Erfindung ist **A** zwischen 60-90cm, vorzugsweise zwischen 65-80 cm, in einer bevorzugten Ausführungsform ist **A** zwischen 70-75 cm lang.

**B** bezeichnet die Gesamtbreite der Liegevorrichtung. In Ausführungsformen der Erfindung ist **B** zwischen 20-50cm, vorzugsweise zwischen 25-40 cm, in einer bevorzugten Ausführungsform ist **B** zwischen 30-35 cm lang.

**C** bezeichnet die Gesamthöhe der Liegevorrichtung (1). In Ausführungsformen der Erfindung ist **C** zwischen 10-40 cm, vorzugsweise zwischen 15-35 cm, in einer bevorzugten Ausführungsform ist C zwischen 20-30 cm hoch.

**D** gibt die Breite der Wand (Wandstärke) der Liegevorrichtung (1) an. In Ausführungsformen der Erfindung ist **D** zwischen 1-10 cm, vorzugsweise zwischen 1-7 cm, in einer bevorzugten Ausführungsform ist **D** zwischen 2-5 cm dick.

**E** bezieht sich auf die Höhe (Innenhöhe) der Wand der Liegevorrichtung (1), wenn eine Matratzeneinlage eingelegt ist und von der Oberseite der Matratzeneinlage (5) der Liegevorrichtung ausgehend gemessen wird.

Die mindestens eine Polstereinlage (6) besitzt in bestimmten Ausführungsformen die gleiche Höhe **E**, sodass ihre Oberkannte mit der Oberkante der Wand der Liegevorrichtung (1) abschließt, wenn die mindestens eine Polstereinlage in den Zentralbereich (3) der Liegevorrichtung (1) eingesetzt ist und auf der Matratzeneinlage aufsitzt.

In manchen Ausführungsformen hat die mindestens eine Polstereinlage (6) eine Höhe **E**, welche sich auf eine Matratzeneinlage mit großer Dicke bezieht, sodass die mindestens eine Polstereinlage auch bei Verwendung einer dicken Matratze nicht über den Rand der Liegevorrichtung hinausragt.

In Ausführungsformen der Erfindung ist **E** zwischen 10-40 cm, vorzugsweise zwischen 10- 30 cm, in einer bevorzugten Ausführungsform ist **E** zwischen 15-25 cm hoch.

**F** bezeichnet den Innenabstand (3b) der Innenwände (3a) des Zentralbereiches (3) ohne eingelegte Polstereinlage(n) (6). In Ausführungsformen der Erfindung ist **F** zwischen 10-35 cm, vorzugsweise zwischen 15-30 cm, in einer bevorzugten Ausführungsform ist **F** zwischen 15-25 cm breit.

**G** bezeichnet den Innenabstand der Innenwände (3a) des Zentralbereiches (3) mit eingelegte(n) Polstereinlage(n). Entsprechend kann der Innenabstand oder Innendurchmesser des Zentralbereiches **G** in Ausführungsformen der Liegevorrichtung durch das Einsetzen oder Herausnehmen einer oder mehrerer Polstereinlagen reguliert werden. Die Distanz von **G** erschließt sich in Ausführungsformen der Erfindung aus der Länge von **F** (3b) minus die Summe der Dicke der mindestens einen Polstereinlage(n) (6) **J**. In Ausführungsformen der Erfindung ist **G** zwischen 5-25 cm, vorzugsweise zwischen 7-20 cm, in einer bevorzugten Ausführungsform ist **G** zwischen 10-15 cm breit.

**H** bezeichnet die Länge der Innenwand (3a) des Zentralbereiches (3). In Ausführungsformen der Erfindung ist **H** zwischen 5-30 cm, vorzugsweise zwischen 10-25 cm, in einer bevorzugten Ausführungsform ist **H** zwischen 12-20 cm lang.

**I** bezeichnet die Länge der mindestens einen Polstereinlage, wobei **I** vorzugsweise gleich der Länge **H** der Innenwand (3a) des Zentralbereiches (3) ist.

In einer bevorzugten Ausführungsform besitzt **I** die gleichen Dimensionen wie **H**.

**J** bezeichnet die Dicke der mindestens einen Polstereinlage(n) (6). In Ausführungsformen der Erfindung ist **J** zwischen 1-15 cm, vorzugsweise zwischen 2,5-10 cm, in einer bevorzugten Ausführungsform ist **J** zwischen 3-7,5 cm dick. In manchen Ausführungsformen kommen Polstereinlagen mit gleichen und/oder unterschiedlichen Dicken **J** vor, welche je nach Bedarf miteinander kombiniert und im Zentralbereich der Liegevorrichtung angebracht werden können oder vorhanden sind. Diese Ausführungsformen bieten vielseitige Anpassungsmöglichkeiten und besondere Flexibilität für ein Kind während des Wachstums, oder z.B. je nach Bekleidung des Kindes und/oder verwendeter Decke.

Der Boden der Liegevorrichtung (1) besitzt in Ausführungsformen eine Dicke zwischen 1-10 cm, vorzugsweise zwischen 2-7 cm, in einer bevorzugten Ausführungsform ist der Boden der Liegevorrichtung zwischen 3-6 cm dick. Die Dicke des Bodens kann in spezifischen Ausführungsformen von der Festigkeit oder Stabilität der verwendeten Materialien abhängig sein, sodass in manchen Ausführungsformen in denen ein stabiles und/oder wenig flexibles Material für den Boden verwendet wird, dieser eine geringere Dicke aufweist. In manchen Ausführungsformen besteht der Boden aus mehreren unterschiedlichen Materialien mit unterschiedlichen Dicken und Festigkeiten/Stabilitäten und/oder Eigenschaften.

In einer spezifischen Ausführungsform haben die Teile der erfindungsgemäßen Liegevorrichtung (1) folgende Dimensionen **A**: 73-75 cm, **B**: 33-35 cm, **C**: 20-25 cm, **D**: 4-5 cm, **E**: 13-18 cm, **F**: 19-22 cm, **G**: 11-13 cm, **H**: 15-17 cm, **I:** 15-17 cm, **J**: 3-5 cm, wobei der Boden der Liegevorrichtung 3-5 cm dick ist.

In einer weiteren spezifischen Ausführungsform haben die Teile der erfindungsgemäßen Liegevorrichtung (1) folgende Dimensionen **A**: 74 cm, **B**: 34 cm, **C**: 20 cm, **D**: 3 cm, **E**: 14 cm, **F**: 20 cm, **G**: 12 cm, **H**: 16 cm, **I:** 16 cm, **J**: 4 cm, wobei der Boden der Liegevorrichtung 3 cm dick ist.

Entsprechend ist die erfindungsgemäße Liegevorrichtung in einer spezifischen Ausführungsform in den Außenmaßen (**A**) 74 cm lang und (**B**) 34 cm breit. Die Seitenwände dieser Ausführungsform der Liegevorrichtung (**C**) sind 20 cm hoch und (**D**) 3 cm dick. Am unteren Rand befindet sich ein 3 cm dicker Boden. Nach oben hin ist die Liegevorrichtung offen, so dass ein Kind hineingelegt werden kann. Der "taillierte" Zentralbereich der Liegevorrichtung ist auf der Innenseite beidseitig durch jeweils eine Polstereinlage, verstärkt. Die beiden Polstereinlagen sind zur Innenseite hin gerade geschnitten, so dass zwei "Schienen" entstehen, in die der Rumpf des Kindes in Seitenlage fixiert wird. In den Innenmaßen ist diese spezifische Ausführungsform 68 cm lang und 28 cm breit, im Zentralbereich zwischen den beiden Polstereinlagen (**G**) 20 cm breit.

In Ausführungsformen der erfindungsgemäßen Liegevorrichtung können sämtliche Zahlenangaben und Bereiche mit +/- 25%, +/- 20%, +/- 10%, +/- 5%, zu verstehen sein.

In Ausführungsformen können auch neue Zahlenbereiche von Dimensionen aus den bestehenden Werten gebildet werden, was durch einen Fachmann routinemäßig durchgeführt werden kann.

In einer weiteren Ausführungsform der erfindungsgemäßen Liegevorrichtung (1) ist die Liegevorrichtung ganz oder teilweise aus einem Material ausgewählt aus Kunststoff, Schaumstoff und/oder Naturstoff gefertigt.

In Ausführungsformen der Erfindung umfassen die Materialien "Kunststoff, "Schaumstoff und "Naturstoff für die Fertigung der erfindungsgemäßen Liegevorrichtung, ohne auf diese begrenzt zu sein, Materialien ausgewählt aus der Gruppe umfassend Kunststoff, Plastik, Polyester, Polyurethan, PET, Schaumstoff, Bio-Kunststoff, Naturstoffe, pflanzen-basierte Kunststoffe, pflanzen-basierte Schaumstoffe, Holz, Holz-Fasern, Naturfasern, Leinen, Baumwolle, Flachs, Jute, Hanf, Seide, Wolle, Fell, Leder, Kunst-Leder, pflanzen-basiertes Kunstleder, Samt, Velours, Fleece, Materialien basierend auf einem oder mehreren der aufgezählten Materialien und/oder jede mögliche Kombination dieser Materialien. Bei den Materialien handelt es sich vorzugsweise um vorwiegend schadstofffreie und/oder Bio-zertifizierte Materialien. Auch die Verwendung von recycelten Materialien, welche vorzugsweise schadstofffrei sind, wird erwägt.

Der Boden der Liegevorrichtung ist in manchen Ausführungsformen aus einem stabileren, festeren und/oder dickerem Material als die Wände gefertigt, sodass er der Liegevorrichtung Stabilität gibt. In bestimmten Ausführungsformen ist auf der Außenseite des Bodens ein wasser- und/oder schmutzabweisendes oder wasserfestes Material oder Beschichtung aufgebracht, welches die Unterseite der Liegevorrichtung vor Verschmutzung schützt. In manchen Ausführungsformen können auch zusätzliche Füße angebracht sein, um den direkten Kontakt der Unterseite der Liegevorrichtung mit dem Boden zu verhindern.

In einer weiteren Ausführungsform der erfindungsgemäßen Liegevorrichtung (1) ist die Liegevorrichtung (1) für die Verwendung als Tragetasche konfiguriert. Erfindungsgemäß besitzen diese Ausführungsformen einen Zentralbereich (3), einen Kopfbereich (2) und einen Fußbereich (4). In diesen Ausführungsformen sollte der Rand der Liegevorrichtung durchgehend gestaltet sein.

In einer spezifischen Ausführungsform verfügt die Liegevorrichtung (1) zusätzlich über eine Tragefunktion, z.B. Tragegriffe, Schultergurt(e) und kann somit zusätzlich auch als Trage- oder Transportvorrichtung bzw. als Tragetasche für Kinder verwendet werden. Erfindungsgemäß besitzen diese Ausführungsformen einen Zentralbereich (3), einen Kopfbereich (2) und einen Fußbereich (4). In diesen Ausführungsformen sollte der Rand der Liegevorrichtung durchgehend gestaltet sein.

In einer weiteren Ausführungsform umfasst die Erfindung eine (separate) Tasche oder Tragetasche, welche die erfindungsgemäße Liegevorrichtung umschließt, und somit die Tragbarkeit der Liegevorrichtung ermöglicht. In manchen solcher Ausführungsformen können sowohl Ausführungsformen der Liegevorrichtung umfassend einen Zentralbereich, einen Kopfbereich und ein Fußbereich, als auch Ausführungsformen der Liegevorrichtung ohne Fuß- und/oder Kopfbereich, oder Ausführungsformen der Liegevorrichtung die am Kopf- und/oder Fußbereich einen offenen/unterbrochenen/keinen Rand besitzen, verwendet werden. In einer Ausführungsform dieser Variante umschließen zwei Gurte jeweils den Zentralbereich der Liegevorrichtung und münden, in einer spezifischen Ausführungsform, in vier Ösen unterhalb des Randes der Tasche. An diesen Ösen können Tragegurte befestigt werden, so dass die erfindungsgemäße Liegevorrichtung wie eine Tragetasche verwendet und getragen werden kann. Andere Ausführungsformen von Tragegurten sind ebenfalls möglich.

In einer weiteren Ausführungsform der erfindungsgemäßen Liegevorrichtung ist die Form der Liegevorrichtung (1) durch den geringeren Durchmesser des Zentralbereichs (3) konfiguriert, um bei der Verwendung als Tragetasche eine Aussparung für die Hüfte des Trägers bereitzustellen.

Die "taillierte" Form von Ausführungsformen der erfindungsgemäßen Liegevorrichtung, welche z.B. an eine Acht oder Sanduhr erinnern kann, ermöglicht es, dass sich die Liegevorrichtung beim Tragen mit der der "Ausbuchtung" des schmaleren Zentralbereiches an die Hüfte des Trägers anschmiegen kann. Hierdurch wird die erfindungsgemäße Liegevorrichtung beim Tragen an der Hüfte des Trägers fixiert. In einer Ausführungsform können optional vorhandene Tragegurte der erfindungsgemäßen Liegevorrichtung zusätzlich in ihrer Länge so angepasst werden, dass die Fixierung auf der Hüfte der tragenden Person optimal angepasst werden kann, was den vorteilhaften Effekt eines erhöhten Tragekomforts bewirkt. In der Ausführungsform der Liegevorrichtung als Tragetasche verbindet die erfindungsgemäße Liegevorrichtung erhöhten Schlafkomfort für Kinder mit einem erhöhten Tragekomfort für die tragende Person, z.B. Eltern.

In einer weiteren Ausführungsform der erfindungsgemäßen Liegevorrichtung (1) ermöglichen die Dimensionen der Liegevorrichtung (1) eine Verwendung als Tragetasche oder als herausnehmbarer Einsatz einer handelsüblichen Tragetasche, eines handelsüblichen Kinderwagens oder einer handelsüblichen Kinderkarre ermöglichen.

In spezifischen Varianten dieser Ausführungsform sind die Dimensionen die Liegevorrichtung spezifisch für die Dimensionen eines bestimmten Kinderwagens oder einer bestimmten Kinderkarre angepasst.

### Optionale medizinische Verwendungen

In einem weiteren Aspekt bezieht sich die Erfindung auf die erfindungsgemäße Liegevorrichtung (1) zur Verwendung bei der Behandlung oder Vorbeugung einer Hinterkopfabflachung (Plagiozephalie) bei Neugeborenen oder Säuglingen. Eine entsprechende medizinische Verwendung kann für spezifische Patienten anwendbar sein, z.B. für Neugeborene oder Säuglinge mit einer Hinterkopfabflachung.

In Ausführungsformen der erfindungsgemäßen Verwendung ermöglicht die Liegevorrichtung (1) eine stabile Seitenlagerung eines Kindes, wobei die Form einen Zentralbereich (3), und mindestens einen Kopfbereich (2), und/oder einen Fußbereich (4) umfasst, wobei der Zentralbereich einen geringeren Durchmesser als der Kopf- (2) und der Fußbereich (4) besitzt.

In Ausführungsformen der Verwendung der erfindungsgemäßen Liegevorrichtung bei der Behandlung oder Vorbeugung einer Hinterkopfabflachung ermöglicht die Liegevorrichtung eine stabile Seitenlagerung eines Kindes, wobei die Form einen Zentralbereich (3), und mindestens einen Kopfbereich (2), und/oder einen Fußbereich (4) umfasst, wobei der Zentralbereich einen geringeren Durchmesser als der Kopf- (2) und der Fußbereich (4) besitzt, und wobei die Form vorzugsweise mindestens eine Polstereinlage (6) enthält, welche an mindestens einer Innenwand (3a) im Zentralbereich (3) angebracht werden kann, um den Durchmesser (3b) des Zentralbereichs (3) zu verringern. In Ausführungsformen der erfindungsgemäßen Verwendung ermöglicht die Liegevorrichtung (1) eine stabile Seitenlagerung eines Kindes, wobei die Form einen Kopfbereich (2), einen Zentralbereich (3) und einen Fußbereich (4) umfasst, wobei der Zentralbereich einen geringeren Durchmesser als der Kopf- (2) und der Fußbereich (4) besitzt.

In Ausführungsformen der erfindungsgemäßen Verwendung enthält die Liegevorrichtung mindestens eine Polstereinlage (6), welche an mindestens einer Innenwand (3a) im Zentralbereich (3) angebracht werden kann, um den Durchmesser (3b) des Zentralbereichs (3) zu verringern.

In Ausführungsformen der Verwendung enthält die Liegevorrichtung mindestens eine Matratzeneinlage (5), wobei vorzugsweise die mindestens eine Matratzeneinlage (5) im Kopfbereich (7) dicker ist als im Zentral- (8) und Fußbereich (9) oder wobei die Matratzeneinlage (5) eine gleichmäßige Dicke besitzt und ein separates Kissen umfasst.

In Ausführungsformen der erfindungsgemäßen Verwendung der Liegevorrichtung (1) sind der geringere Durchmesser des Zentralbereichs (3) und/oder die mindestens eine Polstereinlage (6) im Zentralbereich (3) konfiguriert, um eine Bauch- oder Rückenlage des Kindes zu verhindern.

In Ausführungsformen der erfindungsgemäßen Verwendung der Liegevorrichtung (1) ist das Kind vorzugweise ein Neugeborenes oder ein Säugling.

Wie bereits hierin zuvor beschrieben, erzielt die erfindungsgemäße Liegevorrichtung den überraschenden Effekt, dass durch die Kombination eines großen Luftvolumens im Kopfbereich und der fixierenden Taillierung der Liegevorrichtung im Zentralbereich, eine stabilisierte Seitenlage bewirkt wird. Im Rahmen von spezifischen Ausführungsformen kann die erfindungsgemäße Liegevorrichtung auch in einem medizinischen, präventiven und/oder therapeutischen Kontext verwendet werden. Besonders in diesem Kontext kann in Ausführungsformen durch die Kombination eines großen Luftvolumens im Kopfbereich und der fixierenden Taillierung der Liegevorrichtung im Zentralbereich, welche eine stabilisierte Seitenlage bedingt, zusätzlich zum gesteigerten Wohlbefinden des Kindes der überraschende Effekt erzielt werden, dass die Wahrscheinlichkeit des plötzlichen Kindstods durch eine Bauchlage des Kindes reduziert wird, während gleichzeitig eine Abflachung des Hinterkopfes durch eine Rückenlage des Kindes verhindert wird. Dieser Effekt hat besonders bei der Verwendung der vorliegenden Erfindung in der Behandlung oder Vorbeugung einer Hinterkopfabflachung einen großen Vorteil gegenüber der üblichen Rückenlagerung bzw. nachträglichen Helmtherapie, da zum einen im Falle einer bereits bestehenden Plagiozephalie durch eine Lagerungstherapie mittels der erfindungsgemäßen Liegevorrichtung eine schmerzhaft und stark einschränkende Helmtherapie für Kinder vermieden werden kann. Zum anderen kann bei einer Verwendung der erfindungsgemäßen Liegevorrichtung bereits ab Geburt des Kindes die Rückenlagerungs-bedingte Hinterkopfabflachung erfolgreich verhindert und somit auch die späteren Folgen einer Plagiozephalie vermieden werden.

Somit stellt die vorliegende erfindungsgemäße Liegevorrichtung eine deutliche Verbesserung gegenüber dem Stand der Technik da, da sie eine sichere, komfortable und unkomplizierte Möglichkeit der Seitenlagerung von Kindern ab der Geburt ermöglicht, welche mit den bisher zur Verfügung stehenden Mitteln nicht möglich war.

### DETAILLIERTE BESCHREIBUNG DER ERFINDUNG

Alle zitierten Dokumente der Patent- und Nichtpatentliteratur sind hiermit in ihrer Gesamtheit durch Bezugnahme in die vorliegende Offenbarung aufgenommen.

Die vorliegende Erfindung bezieht sich auf eine Liegevorrichtung, welche durch ihre Form eine stabile Seitenlagerung eines Kindes ermöglicht.

Der Begriff "Kind" umfasst im Kontext der vorliegenden Erfindung die folgenden Altersgruppen: Neugeborene bis zum vollendeten 28. Lebenstag, Säuglinge ab Beginn des 29. Lebenstages bis zum vollendeten 12. Lebensmonat, Kleinkinder ab Beginn des 2. bis zum vollendeten 3. Lebensjahr und/oder Kinder ab Beginn des 4. bis zum vollendeten 12. Lebensjahr. Vorzugsweise bezieht sich der Begriff Kind auf Kinder bis zum Beginn des 4. Lebensjahres, welche eine Körpergröße/Länge von maximal 90 cm besitzen. Im Allgemeinen kann im Kontext der vorliegenden Erfindung der Begriff "Kind" als Oberbegriff für alle Personen unter 12 Jahren, vorzugsweise unter 4 Jahren verwendet werden.

Als "plötzlichen Kindstod" oder "plötzlichen Säuglingstod" (SIDS: Sudden Infant Death Syndrome) bezeichnet man das unerwartete, plötzliche Versterben eines Neugeborenen oder Säuglings vor seinem 365. Lebenstag. Die Todesursache kann in der Regel trotz gründlicher Untersuchung nicht bzw. nicht eindeutig geklärt werden. Meist versterben die Kinder nachts im Schlaf - Jungs häufiger als Mädchen. Die Mehrzahl der SIDS Betroffenen wird dabei von ihren Eltern in Bauchlage aufgefunden, das Schlafen in der Bauchlage ist daher mit einem erhöhten Risiko für SIDS assoziiert. Entsprechend empfehlen Ärzte das Schlafen in der Rückenlage für Kinder im ersten Lebensjahr, da diese sich anfangs noch nicht selbstständig drehen können, sodass eine Rückenlagerung das Hinüberrollen in die Bauchlage verhindert. Obwohl von vielen Kindern zum Schlafen bevorzugt, wird die Seitenlage nicht als Schlafposition empfohlen, da sie von Neugeborenen und Säuglingen nicht stabil gehalten werden kann, sodass die Gefahr des Rollens in die risikoassoziierte Bauchlage besteht.

"Plagio-zephalus" oder "Plagiozephalie" ist eine asymmetrische Abflachung des Hinterkopfes, wobei das Verhältnis Länge zu Breite des Schädels nicht verändert ist. Längeres Liegen in der Rückenlage kann die Form des noch formbaren Schädels von Säuglingen am Hinterkopf beeinflussen und zur Plagiozephalie führen. Das Gehirn wird durch die Form der Schädelkalotte nicht beeinflusst. Im Zusammenhang mit vorzeitigem Nahtverschluss kann es auch zu Gesichtsasymmetrien kommen. Die von Ärzten empfohlene Rückenlage bei Säuglingen führt zu einem deutlichen Rückgang des plötzlichen Kindstodes, begünstigt jedoch auch die lagerungsbedingte Kopfverformung.

Die Begriffe Matratzeneinlage und Matratze sind im Kontext der vorliegenden Erfindung austauschbar. Ausführungsformen der vorliegenden Erfindung kann eine Matratze oder Matratzeneinlage auch ein Kopfkissen umfassen.

### FIGUREN

Die Erfindung wird durch die folgenden Figuren weiter beschrieben. Diese sollen den Umfang der Erfindung nicht einschränken, sondern stellen bevorzugte Aspekte der Erfindung dar, die zur näheren Veranschaulichung der beschriebenen Erfindung offenbart werden.

### Kurzbeschreibung der Figuren:

**Figur 1****:** Schema der erfindungsgemäßen Liegevorrichtung mit darin liegendem Kind in der Seitenlage (A) und mit angebrachten Polstereinlagen im Zentralbereich (B).
**Figur 2****:** Dimensionen einer spezifischen Ausführungsform der Erfindung.
**Figur 3****:** Verschiedene Ausführungsformen der erfindungsgemäßen Liegevorrichtung in der Aufsicht.
**Figur 4****:** Verschiedene Ausführungsformen der erfindungsgemäßen Liegevorrichtung in der Aufsicht. Vergleichsansicht der Liegevorrichtung mit und ohne eingelegte Matratze.
**Figur 5****:** 3-dimensioanle Darstellung der in den Figuren 3 und 4 dargestellten Ausführungsformen der Liegevorrichtung (1) und der Matratze (5), Kopfkissen (7/5) und Polstereinlagen (6).
**Figur 6****:** 3-dimensioanle Darstellung der in Figur 3 und 4 dargestellten Liegevorrichtung (1) mirt eingelegter Matratze (5), einem im Kopfbereich (2) eingelegten zusätzlichen Kopfkissens (7) und zwei im Zentralbereich (3) angebrachten Polstereinlagen (6).

### Detaillierte Beschreibung der Figuren:

**Figur 1****:** Schema beispielhafter Ausführungsformen der erfindungsgemäßen Liegevorrichtung (1) mit A) darin liegendem Kind in der Seitenlage und B) mit angebrachten Polstereinlagen im Zentralbereich. Abbildung A) zeigt Kopf- (2), Zentral- (3) und Fußbereich (4) der Liegevorrichtung (1). Wie A) dargestellt liegt das Kleinkind mit dem Torso im verengten Zentralbereich (3), wobei die Seitenlage des Kindes stabilisiert wird, und das Kind ein Gefühl von Widerstand und Stabilisierung im Torso-Bereich erfährt. Dieses Gefühl von Stabilisierung/Unterstützung zusammen mit dem Liegen in der Seitenlage kann sich beruhigend und schlaffördernd auf das

Kind auswirken. Zudem wird eine Bauch- und Rückenlage, und die damit verbundenen Risiken und Nachteile, wirksam verhindert. Die stabilisierende Begrenzung im Zentralbereich (3) kann durch eine oder mehrere Polstereinlagen (6), welche im Zentralbereich (3) angebracht werden können, verbessert und individuell auf das Kind angepasst werden, siehe B). Die Proportionen der erfindungsgemäßen Liegevorrichtung und besonders des Zentralbereiches können in Ausführungsformen der Erfindung von der Darstellung abweichen.

**Figur 2****:** Dimensionen einer spezifischen Ausführungsform der Erfindung. **A** bezieht sich auf die Gesamtlänge der Liegevorrichtung (1), **B** die Gesamtbreite und **C** die Gesamthöhe der Liegevorrichtung (1). **D** gibt die Breite der Wand (Wandstärke) der Liegevorrichtung (1) an und **F** bezeichnet den Innenabstand (3b) der Innenwände (3a) ohne eingelegte Polstereinlage(n) (6). **E** bezieht sich auf die Höhe (Innenhöhe) der Wand der Liegevorrichtung (1), wenn von der Oberseite der Matratzeneinlage (5) der Liegevorrichtung ausgehend gemessen wird, wobei die mindestens eine Polstereinlage (6) vorzugsweise die gleiche Höhe **E** besitzt, sodass ihre Oberkannte mit der Oberkante der Wand der Liegevorrichtung (1) abschließt, wenn die mindestens eine Polstereinlage in den Zentralbereich (3) der Liegevorrichtung (1) eingesetzt ist. **G** bezeichnet den Innenabstand der Innenwände (3a) mit eingelegte(n) Polstereinlage(n). Die Distanz von **G** erschließt sich aus der Länge von **F** (3b) minus die Summe der Dicke der mindestens einen Polstereinlage(n) (6) **J**. **I** bezeichnet die Länge der mindestens einen Polstereinlage, wobei **I** vorzugsweise gleich der Länge **H** der Innenwand (3a) des Zentralbereiches (3) ist.

**Figur 3****:** Verschiedene Ausführungsformen der erfindungsgemäßen Liegevorrichtung in der Aufsicht. A) zeigt die leere Liegevorrichtung (1) mit Kopf- (2), Zentral- (3) und Fußbereich (4) ohne Matratze, Kissen oder Polstereinlagen. B) zeigt Ausführungsformen des Kopfkissens in verschiedenen Materialdicken (7/5) sowie keilförmige Ausführungsformen der Polstereinlagen (6). C) zeigt eine Matratzeneinlage (5), welche einen Kopfbereich (7), einen Zentral-(8) und einen Fußbereich (9) umfasst, wobei der Kopfbereich (7) eine andere Dicke, als der Zentral- (8) und der Fußbereich (9) hat.

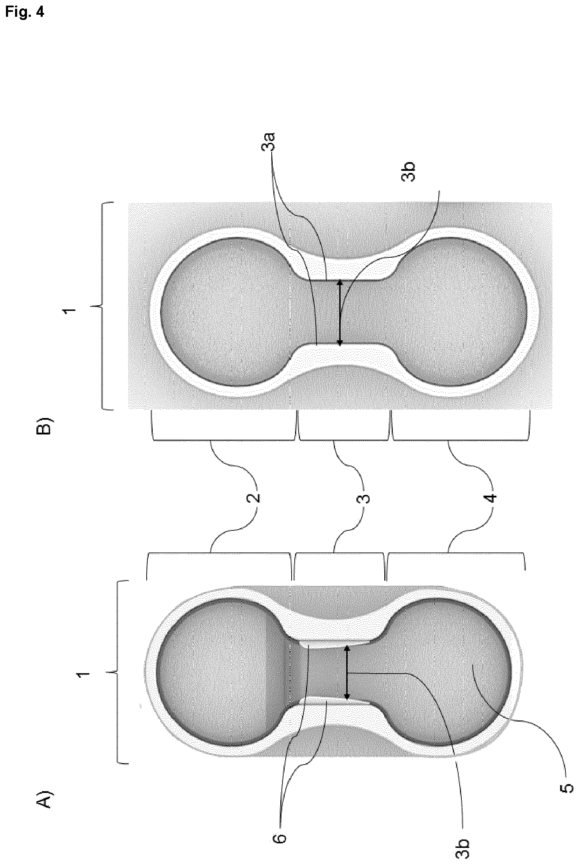

**Figur 4****:** Verschiedene Ausführungsformen der erfindungsgemäßen Liegevorrichtung in der Aufsicht. Vergleichsansicht der B) leeren Liegevorrichtung (1) mit Kopf- (2), Zentral- (3) und Fußbereich (4) und A) der Liegevorrichtung (1) mit eingelegter Matratze (5). Die Abbildung A) illustriert wie die in Figur 3B und C exemplarisch dargestellten Matratze, Kopfkissen und Polstereinlagen in die Liegevorrichtung (1) eingelegt werden können. Der Durchmesser (3b) des Zentralbereiches (3) kann durch die Anbringung von Polstereinlagen (6) an die Innenwände (3a) verringert werden (siehe linke Abbildung). Durch ein zusätzlich im Kopfbereich (2) eingelegtes Kopfkissen oder eine Matratze (5) welche im Kopfbereich (7) dicker ist, als im Fuß- (8) und Zentralbereich (9) kann der Liegekomfort des Kindes erhöht werden, da der Kopf in der Seitenlage stabilisiert und der Nacken und der unten liegende Arm entlastet werden.

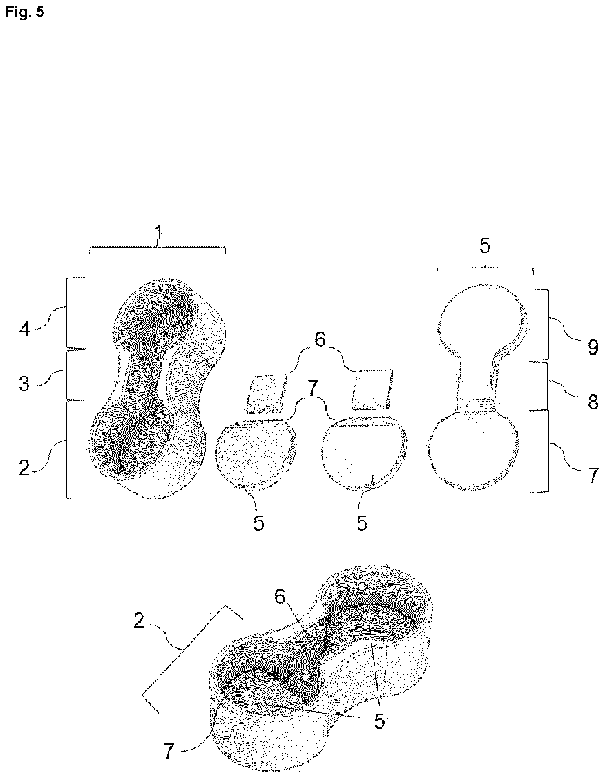

**Figur 5****:** 3-dimensioanle Darstellung der in Figur 3 und 4 dargestellten Ausführungsformen der Liegevorrichtung (1) und der Matratze (5), Kopfkissen (7/5) und Polstereinlagen (6).

**Figur 6****:** 3-dimensioanle Darstellung der in Figur 3 und 4 dargestellten Liegevorrichtung (1) mit eingelegter Matratze (5), im Kopfbereich (2) eingelegtes zusätzliches Kopfkissen (7) und zwei an der Innenwand (3a) des Zentralbereichs (3) angebrachten Polstereinlagen (6).

### BEISPIELE

Die Erfindung wird durch die Beispiele weiter charakterisiert. Diese sollen den Umfang der Erfindung nicht einschränken, sondern stellen bevorzugte Ausführungsformen von Aspekten der Erfindung dar, die zur besseren Veranschaulichung der hierin beschriebenen Erfindung bereitgestellt werden.

Eine beispielhafte Darstellung der Erfindung findet sich in den Figuren 1-6.

In einem Ausführungsbeispiel sind die in Figur 2 bezeichneten Dimensionen wie folgt:
Die Liegevorrichtung (1) besitzt die folgenden Außenmaße: Sie ist (**A**) 74 cm lang und (**B**) 34 cm breit. Die Seitenwände dieser Ausführungsform der Liegevorrichtung (**C**) sind 20 cm hoch und (**D**) 3 cm dick. Am unteren Rand befindet sich ein 3 cm dicker Boden. Nach oben hin ist die Liegevorrichtung offen, so dass ein Kind hineingelegt werden kann. Der "taillierte" Zentralbereich der Liegevorrichtung ist auf der Innenseite beidseitig durch jeweils eine Polstereinlage (6) verstärkt. Die beiden Polstereinlagen (6) sind zur Innenseite hin gerade geschnitten, so dass zwei stabilisierende "Schienen" entstehen, die den Rumpf des Kindes in Seitenlage fixieren. In den Innenmaßen ist diese spezifische Ausführungsform 68 cm lang und 28 cm breit, im Zentralbereich zwischen den beiden Polstereinlagen (3b, **G**) 20 cm breit. Die weiteren Maße betragen für die Höhe der Polstereinlagen (6) **E**: 14 cm. Der Zentralbereich (3) ist innen ohne Polstereinlagen, **F**: 20 cm und mit Polstereinlagen **G**: 12 cm breit/weit, wobei der Zentralbereich (3) ohne Polstereinlagen **H**: 16 cm lang ist. Die Polstereinlagen (6) haben an der Außenkante, mit welcher Sie an dem Zentralbereich (3a) fixiert werden, ebenfalls eine Länge von **I:** 16 cm. Die Dicke der Polstereinlagen (6) beträgt **J**: 4 cm.

## Patentansprüche

1. Liegevorrichtung (1), welche durch ihre Form eine stabile Seitenlagerung eines Kindes ermöglicht, wobei
die Form einen Zentralbereich (3), und mindestens einen Kopfbereich (2), und/oder einen Fußbereich (4) umfasst, wobei der Zentralbereich einen geringeren Durchmesser als der Kopf- (2) und der Fußbereich (4) besitzt.

2. Liegevorrichtung (1) gemäß Anspruch 1, wobei die Form mindestens eine Polstereinlage (6) enthält, welche an mindestens einer Innenwand (3a) im Zentralbereich (3) angebracht werden kann, um den Durchmesser (3b) des Zentralbereichs (3) zu verringern.

3. Liegevorrichtung (1) gemäß einem der vorhergehenden Ansprüche, wobei der geringere Durchmesser des Zentralbereichs (3) und/oder die mindestens eine Polstereinlage (6) im Zentralbereich (3) konfiguriert sind, um eine Bauch- oder Rückenlage des Kindes zu verhindern.

4. Liegevorrichtung (1) gemäß einem der vorhergehenden Ansprüche, wobei die Liegevorrichtung (1) mindestens eine Matratzeneinlage (5) umfasst.

5. Liegevorrichtung (1) gemäß einem der vorhergehenden Ansprüche, wobei die Liegevorrichtung ganz oder teilweise aus einem Material ausgewählt aus Kunststoff, Schaumstoff und/oder Naturstoff gefertigt ist.

6. Liegevorrichtung (1) gemäß einem der vorhergehenden Ansprüche, wobei das Kind vorzugweise ein Neugeborenes oder ein Säugling ist.

7. Liegevorrichtung (1) gemäß einem der Ansprüche 4-6, wobei die mindestens eine Matratzeneinlage (5) aus einem verbundenen Teil oder aus mehreren separaten oder verbundenen Teilen besteht, umfassend ein Zentralteil (8), und mindestens ein Kopfteil (7), und/oder ein Fußteil (9), wobei die separaten oder verbundenen Teile der Matratzeneinlage (5) eine unterschiedliche oder die gleiche Dicke aufweisen.

8. Liegevorrichtung (1) gemäß einem der vorhergehenden Ansprüche, wobei die Form einen Zentralbereich (3), einen Kopfbereich (2), und einen Fußbereich (4) umfasst.

9. Liegevorrichtung (1) gemäß Anspruch 8, wobei die Liegevorrichtung (1) die Dimensionen (Breite x Länge) von 25-50 x 60-100 cm besitzt, vorzugsweise von 30-40 x 65-85 cm, wobei die Außenwände der Liegevorrichtung (1) zwischen 15-30 cm hoch sind, vorzugsweise zwischen 20-35 cm hoch.

10. Liegevorrichtung (1) gemäß einem der Ansprüche 8 oder 9, wobei die Liegevorrichtung (1) für die Verwendung als Tragetasche konfiguriert ist.

11. Liegevorrichtung (1) gemäß Anspruch 10, wobei die Form der Liegevorrichtung (1) durch den geringeren Durchmesser des Zentralbereichs (3) konfiguriert ist, um bei der Verwendung als Tragetasche eine Aussparung für die Hüfte des Trägers bereitzustellen.

12. Liegevorrichtung (1) gemäß einem der vorhergehenden Ansprüche, wobei die Dimensionen der Liegevorrichtung (1) eine Verwendung als Tragetasche oder als herausnehmbarer Einsatz einer handelsüblichen Tragetasche, eines handelsüblichen Kinderwagens oder einer handelsüblichen Kinderkarre ermöglichen.

13. Liegevorrichtung (1) gemäß einem der Ansprüche 1-7, wobei der Rand der Liegevorrichtung eine unterschiedliche Höhe im Zentralbereich (3), Kopfbereich (2) und/oder Fußbereich (4) besitzt oder im Kopf- und/oder Fußbereich unterbrochen ist.
